# EUROPEAN PATENT APPLICATION

(11) **EP 4 335 480 A1**
(43) Date of publication of application: **13.03.2024**
(21) Application number: 21865360.8
(22) Date of filing: 13.08.2021
(51) Int. Cl.: A61M 25/10, A61B 17/22

(54) **PULSE BALLOON AND APPLICATION THEREOF**

(30) Priority: 08.05.2021 CN 202110498081
(71) Applicant: Shanghai Bio-Heart Biological Technology Co., Ltd., Shanghai 201201 (CN)
(72) Inventor: WANG, Philip Li, Shanghai 200120 (CN); ZHANG, Chenzhao, Shanghai 200120 (CN); LIANG, Xinfeng, Shanghai 200120 (CN); CAI, Tao, Shanghai 200120 (CN); WANG, Junyi, Shanghai 200120 (CN)
(74) Representative: Jannig & Repkow Patentanwälte PartG mbB
(86) International application number: PCT/CN2021/112371
(87) International publication number: WO 2022/236989

(57) **Abstract**

The present invention provides a medical instruments, more particularly, provides a pulse balloon and use thereof. The pulse balloon comprises a balloon body (1) and an inner tube (2), wherein the balloon body (1) comprises an insulating layer (11) and a balloon wall (12). The insulating layer (11) is arranged on the balloon body (1), when the balloon body operates, the balloon body (1) is filled with an electrolyte so that an electrode releases high-pulse piezoelectricity to generate pulses, the electrolyte spreads to drive the vibration of the balloon, so that most of electrical energy is converted into mechanical energy to break down a calcified area of a blood vessel, and the residual high voltage is blocked by the insulating layer (11) so as to avoid the conduction of high voltage on the surface of the balloon and restrict the excess piezoelectricity at a pulse source. By using the balloon body (1) with the insulating layer (11), excess electricity can be prevent from being conducted to a pacemaker, a signal transduction area, etc., so that the heart rate of a patient can be maintained at a relatively stable state. When facing patients with severe calcification, the discharging voltage and discharging times can be increased, thereby avoiding damage to a human body while promoting the effectiveness of surgery.

## Description

### TECHNICAL FIELD

The present invention relates to the field of medical instruments, more particularly to a pulse balloon and use thereof.

### BACKGROUND

Aortic calcification, also known as arteriosclerosis, is accumulation of calcium deposits on heart. This often causes heart murmur which can be readily heard by a stethoscope putting over the heart. However, aortic calcification often does not distinctly affect the function of aorta valves. But, in some cases, the calcium deposits become thick and allow an opening on the aorta valve to become narrow. This reduces a blood flow volume passing through the valve so as to cause chest pain or heart attack, and such stenosis is called aortic stenosis.

At present, a way to reduce calcification is to apply shock waves to the calcified area of the valve to promote and/or crack calcium deposits, so as to soften and/or loosen and/or remove the calcium deposits of the mechanical properties of the hardened valve. For example, CN104582597A, CN 109223100 A, CN 111184553 A and CN 104519809A and the like all disclose a pulse balloon structure by which the use of pulses drives the vibration of the electrolyte so that the inner wall of the balloon undergoes physical vibration, and the generated physical vibration is opposed to the calcified area of the blood vessel to achieve the purpose of breaking down or shocking the calcified area.

For the current pulse balloon, the pulse spreading through liquid is generated by discharging via a cathode and an anode in the balloon. In the process of discharging, the cathode and the anode generate high voltage electricity, and the wall thickness of the balloon is particularly small. In the conduction through the electrolyte, a part of high voltage electricity can break down the thin wall, and a part of high voltage electricity will appear on the outermost side of the balloon, the high voltage electricity acts on the patient's blood vessels so as to cause the heart rate to be disturbed.

### SUMMARY

In order to solve the above problems, the first aspect of the present invention provides a pulse balloon, the pulse balloon comprising a balloon body and an inner tube, wherein the inner tube penetrates through the balloon body; the balloon body comprises an insulating layer and an inner tube; the insulating layer is located at one side and/or interior of the balloon wall; the balloon wall has a thickness of 10-30 µm, and the insulating layer has a thickness of 1-5 µm;
when the insulating layer is located at one side of the balloon wall, the balloon body is prepared by coating, centrifugation, sleeve blow molding and extrusion blow molding;
when the insulating layer is located inside the balloon wall or when the insulating layer is located at one side and the interior of the balloon, the balloon body is prepared by extrusion blow molding;
the coating comprises: after the balloon wall is subjected to blowing molding, coating an insulating layer material dispersion at one side of the balloon wall, and drying at 150-200°C;
the centrifugation comprises: after the balloon wall is subjected to blowing molding, introducing an insulating layer material dispersion into the interior of the balloon wall, and undergoing centrifugal rotation at 150-200°C;
the sleeve blow molding comprises: sheathing an insulating layer film outside a balloon tube for blow molding;
the extrusion blow molding comprises: co-extruding the insulating layer and the balloon wall to obtain a complex tube for blow molding;
the process parameters of the blow molding are as follows: the temperature is 100-250°C, the heating time is 10-150s, and the pressure is 50-1000psi.

As a preferred embodiment of the present invention, the balloon body comprises the insulating layer and the balloon wall from outside to inside.

As a preferred embodiment of the present invention, the balloon body comprises the balloon wall and the insulating layer from outside to inside.

As a preferred embodiment of the present invention, the insulating layer is located inside the balloon wall.

As a preferred embodiment of the present invention, the inner tube is in seal connection with the balloon body.

As a preferred embodiment of the present invention, the inner tube is located on the symmetry axis of the balloon body.

As a preferred embodiment of the present invention, the inner tube is provided with an electrode.

As a preferred embodiment of the present invention, the material of the balloon wall is selected from one or more of nitrogenous polymers, polyesters and epoxy resins.

As a preferred embodiment of the present invention, the material of the insulating layer is selected from one or more of plastics, rubbers, fibers and inorganic materials.

The second aspect of the present invention provides use of the pulse balloon for shielding electrical interference in a vascular calcification area.

Compared with the prior art, the present invention has the beneficial effects:
(1) The balloon body is provided with the insulating layer, and when the balloon operates, the balloon body is filled with the electrolyte, so that the electrode releases high-pulse piezoelectricity to generate pulses, and the electrolyte spreads to drive the vibration of the balloon so that most of electrical energy is converted into mechanical energy to break down the calcified area of the blood vessel, and the residual high voltage is blocked by the insulating layer so as to avoid the conduction of the high voltage on the surface of the balloon and restrict the excess high voltage electricity at the pulse source.
(2) The use of the balloon body with the insulating layer can prevent excess electricity from being conducted to the pacemaker, signal conduction areas, etc., so that the patient's heart rate can be maintained in a relatively stable state.
(3) In the face of patients with severe calcification, the discharging voltage and the discharging number can be increased so as to avoid damage to the human body while promoting the effectiveness of surgery.
(4) The inner tube is arranged on the axis of the balloon, and the electrode is arranged on the inner tube, which can ensure that the vibration of the pulse transmitted to the surface of the balloon is kept symmetrical so as to avoid that uneven vibration affects the crushing of the calcified area, thereby improving the service life of the balloon body, reducing partial detachment and separation of the insulating layer and the balloon wall.
(5) The insulating layer and the balloon wall are fixed by spraying, sleeve, gluing, blow molding, hot pressing and other ways, so as to improve the bonding degree of the insulating layer and the balloon wall, improve the uniformity of the distribution of the insulating layer, and avoid that the surface of the partial balloon is not covered with the insulating layering, and the inventors found that the balloon obtained by virtue of the interaction between the balloon wall and the insulating layer provided by the present invention can act under the action of high-voltage pulses, thereby avoiding separation of the insulating layer from the balloon wall, and improving the service life.
(6) The balloon body can comprise one or more insulating layers, and when one insulating layer is present, it can play a good role in preventing electrical energy from being blocked.

### BRIEF DESCRIPTION OF DRAWINGS

In order to illustrate the embodiments of the present invention or the technical solutions in the prior art more clearly, the accompanying drawings that are used in the description of the embodiments or the prior art will be briefly introduced below. Obviously, the drawings in the following description are only some embodiments of the present invention. For persons of ordinary skill in the art, other drawings can also be obtained according to these drawings without creative efforts.
Fig. 1 is an example of a structural diagram of a pulse balloon.
Fig. 2 is an example of a structural diagram of a pulse balloon.
Fig. 3 is an example of a structural diagram of a pulse balloon.

In the Figures: 1-balloon body, 2-inner tube, 11-insulating layer, 12-balloon wall.

### DESCRIPTION OF THE EMBODIMENTS

The contents of the present invention can be more easily understood by reference with the detailed description of the preferred implementation methods and included embodiments. Unless otherwise defined, all technical and scientific terms used herein have the same meaning as commonly understood by one of ordinary skill in the art to which this invention belongs. In case of conflict, the definitions in this specification will control.

The singular form includes the plural object of discussion unless the context clearly dictates otherwise. "Optional" or "either" means that the subsequently described item or event may or may not occur, and that the description includes instances where the event occurs and instances where it does not.

Furthermore, the indefinite articles "a" and "an" preceding an element or component of the invention are not limiting on the quantitative requirement (i.e., the number of occurrences) of the element or component. Thus "a" or "an" should be read to include one or at least one, and elements or components in the singular also include the plural unless the number is clearly intended to be in the singular.

In the description of the present invention, it should be noted that the terms "center", "upper", "lower", "left", "right", "vertical", "horizontal", "inner", "outer", etc. The indicated orientation or positional relationship is based on the orientation or positional relationship shown in the accompanying drawings, or the orientation or positional relationship that the product of the invention is usually placed in use, only for the convenience of describing the present invention and simplifying the description, rather than indicating or implying The device or element referred to must have a particular orientation, be constructed and operate in a particular orientation, and therefore should not be construed as limiting the invention. Furthermore, the terms "first", "second", "third" and the like are only used to differentiate the description and should not be construed as indicating or implying relative importance.

Furthermore, the terms "horizontal", "vertical", "overhanging" etc. do not imply that a component is required to be absolutely horizontal or overhang, but rather may be slightly inclined. For example, "horizontal" only means that its direction is more horizontal than "vertical", it does not mean that the structure must be completely horizontal, but can be slightly inclined.

In the description of the present invention, it should also be noted that, unless otherwise expressly specified and limited, the terms "arrangement", "installation", "connection" and "linking" should be understood in a broad sense, for example, fixed connection, detachable connection or integral connection; or mechanical connection or electrical connection; or direct connection or indirect connection through an intermediate medium, or internal communication between two elements. For persons of ordinary skill in the art, the specific meanings of the above terms in the present invention can be understood in specific situations.

The first aspect of the present invention provides a pulse balloon, the pulse balloon comprising a balloon body and an inner tube, wherein the inner tube penetrates through the balloon body; the balloon body comprises an insulating layer and an inner tube, and the insulating layer is located at one side or the interior of the balloon wall.

In one embodiment, the balloon body comprises the insulating layer and the balloon wall from outside or inside. In one embodiment, the balloon body comprises the balloon wall and the insulating layer from outside to inside. In one embodiment, the insulating layer is located inside the balloon wall.

In one embodiment, the inner tube is in seal connection with the balloon body. In one embodiment, the inner tube is located on the symmetry axis of the balloon body. In one embodiment, the inner tube is provided with an electrode.

In one embodiment, the balloon wall has a thickness of 10-30 µm, for example, 10 µm, 15 µm, 20 µm, 25 µm and 30 µm. In one embodiment, the insulating layer has a thickness of 1-5 µm, for example, 1 µm, 2 µm, 3 µm, 4 µm and 5 µm.

The inventor finds that by setting the functions of the insulating layer and the balloon wall, it is beneficial to preventing the high voltage electricity from breaking down the balloon in the process of discharging so as to avoid affecting the heart rate, but at the same time, it is generally necessary to control the thickness of the balloon body to be very small in order to improve the pulse effect, so as to difficultly prepare the insulating layer and the balloon wall which are good in fit to affect the practical use of the balloon, especially in high voltage. However, the inventors find that a high-fit balloon can be prepared by using preparation methods such as spraying, sleeve, gluing and blow molding and selecting proper insulating layer and balloon wall material, cannot be cracked and fallen off even though at high discharging voltage and discharging times, can be used for serious calcified areas, thereby improving the effectiveness of surgery and avoiding damage to a human body. Furthermore, the inventors find that the thicknesses of the insulating layer and the balloon wall are all needed to be controlled within an appropriate range. It is not that the higher the thickness of the insulating layer is, the better is. When the thickness is high, it is easy to cause problems such as cracking. When the thicknesses of the insulating layer and the balloon wall are reasonable, it is also conducive to the smooth progress of the blow molding process to avoid the problem of inconsistent blow molding degree caused by different materials, so it can be used for pulse impact under high voltage and discharging times.

In one embodiment, the material of the balloon wall is one or more selected from the group consisting of nitrogenous polymers, polyesters and epoxy resins, preferably nitrogenous polymers. As an example of nitrogenous polymers, polyethyleneimine, polyamide, aromatic polyamide such as polyparabenzamide and polymetaphenylene isophthalamide, aliphatic polyamide such as polyamide 6 and polyamide 66, and imide such as polyamideimides, polymaleimides are listed; preferably polyvinylamide and polyimide.

In one embodiment, the material of the insulating layer is selected from one or more of plastics, rubbers, fibers and inorganic materials. As an example of plastics, polyolefin such as polypropylene, polyethylene, polyvinyl chloride, polyester, fluorine-containing polyolefin such as polytetrafluoroethylene, polyvinylidene fluoride and polyperfluoroethylene propylene are listed; as an example of rubbers, epoxy resin glue and epoxy polyester glue are listed; as an example of fibers, insulating paper is listed; as an example of inorganic materials, ceramics and glass fibers are listed; preferably plastic or rubber; more preferably fluorinated polyolefin.

In one embodiment, the balloon body is prepared by at least one of coating, centrifugation, sleeve blow molding, and extrusion blow molding. The coating comprises spraying, brushing, and dipping. When the insulating layer is located on one side of the balloon wall, and the balloon body is prepared by coating, sleeve blow molding and extrusion blow molding. In one embodiment, when the balloon body comprises the insulating layer and the balloon wall from outside to inside, the balloon body is prepared by at least one of spraying, brushing, dipping and sleeve blow molding. In one embodiment, when the balloon body comprises the balloon wall and the insulating layer from outside to inside, the balloon body is prepared by at least one of spraying, brushing, dipping, centrifugation and extrusion blow molding. In one embodiment, the balloon body is prepared by extrusion blow molding when the insulating layer is located inside the balloon wall or when the insulating layer is located at one side and the interior of the balloon.

In one embodiment, the coating comprises: after the balloon wall is subjected to blow molding, the insulating layer material dispersion is coated on one side of the balloon wall, and dried at 150-200°C.

Arranging the insulating layer is arranged on the inner surface or outer surface of the balloon wall by spraying or brushing includes: after the balloon wall is subjected to blow molding, spraying or brushing the insulating layer material dispersion on the outer surface and/or outer surface of the balloon wall, and drying at 150-200°C. Wherein, before spraying or brushing, the outer surface/or inner surface of the balloon wall can be subjected to degreasing and tackifying treatments. The degreasing refers to using a cleaning method adopting a solvent such as ethanol and acetone to remove the grease on the outer surface of the balloon wall. The tackifying is mainly to improve the roughness or adhesion performance of the surface of the balloon wall, which can be achieved by sandblasting, sandpaper grinding or spraying an adhesive. The insulating layer material dispersion is a dispersion solution containing insulating layer materials, and can be prepared into a suspending agent by dispersing the insulating layer material into an organic solvent such as ethanol and acetone or water. The insulating layer material dispersion of the present invention, such as polytetrafluoroethylene dispersion, can be self-made or purchased. In addition, powder spraying can be used for spraying, including: insulating layer material powder is sprayed onto the outer surface and/or inner surface of the balloon wall, and then cured by heating, wherein prior to powder spraying, the outer surface of the balloon wall can also be subjected to degreasing and tackifying treatments.

Arranging the insulating layer on the inner surface or outer surface of the balloon wall by dipping includes: after blow molding of the balloon wall, immersing the outer surface/or inner surface of the balloon wall into the insulating layer material dispersion, then taking out and drying at 150-200°C. According to the present invention, before dipping, adhesive treatment can be performed, for example, the outer surface/or inner surface zion of the balloon wall is dipped into the adhesive, the balloon wall is taken out or the adhesive is discharged, and then immersion is performed in the insulating layer material dispersion; the balloon wall is taken out or the insulating layer material dispersion, and then drying is performed at 150-200°C. Wherein, the immersion in the insulating layer material dispersion can be performed for 15-30 min.

Arranging the insulating layer on the inner surface of the balloon wall by centrifugation includes: after blow molding of the balloon wall, introducing the insulating layer material dispersion into the interior of the balloon wall, performing centrifugal rotation at 150-200°C so that the dispersion is evenly distributed on the inner wall around the balloon under the action of a centrifugal force, stopping the rotation after complete drying, and removing. The centrifugal rate is 800-10000 rpm, preferably 800-5000 rpm, more preferably 800-2000 rpm. The inventors find that the insulating layer dispersion is dispersed onto the surface of the balloon wall by spraying, brushing, dipping, centrifugation and other manners and dried at a certain temperature, so as to form an even insulating layer for use in the pulse balloon.

Fixing the insulating layer material onto the outer surface of the balloon wall by sleeve blow molding includes: sleeving the insulating layer film on the outer layer of the balloon tube, and putting into a mold of a balloon molding machine for blow molding. Fixing the insulating layer material onto the outer or inner surface or the interior of the balloon wall by extrusion blow molding includes: extruding the insulating layer and the balloon wall into a complex tube with a tube extruder by outer diameter sizing, and putting the complex tube into the mold of the balloon molding machine for blow molding. The inventors find that, for sleeve blow molding or extrusion blow molding, a method of forming the tube and then blow molding is adopted, which is beneficial to the bonding between the insulating layer and the balloon wall.

In one embodiment, the process parameters for blow molding are: the blow molding temperature is 110-250°C, preferably 110-200°C, more preferably 110-150°C; the heating time is 10-150s, preferably 10 -100s, more preferably 10-50s, more preferably 10-25s, more preferably 10-15s; the pressure is 50-1000psi, preferably 50-500psi, more preferably 50-300psi, more preferably 50- 100 psi, more preferably 50-70 psi. In one embodiment, the tensile speed of the left and right sides of the mold is 120-250 mm/s, preferably 120-200 mm/s, more preferably 120-180 mm/s, more preferably 120-170 mm/s; the cooling time of the left and right sides is 5-100s, preferably 5-80s, more preferably 5-50s, more preferably 5-20s, more preferably 5-10s. The left and right sides of the mold correspond to the two sides connecting the balloon with the inner tube.

The second aspect of the present invention provides use of the above-mentioned pulse balloon for shielding electrical interference in a vascular calcification area.

### Examples

The present invention will be specifically described through examples below. It is necessary to point out that the following examples are only used to further illustrate the present invention, and should not be construed as limiting the scope of protection of the present invention, and some non-essential improvements made by those skilled in the art according to the above-mentioned content of the present invention and adjustment, still belong to the protection scope of the present invention.

### Example 1

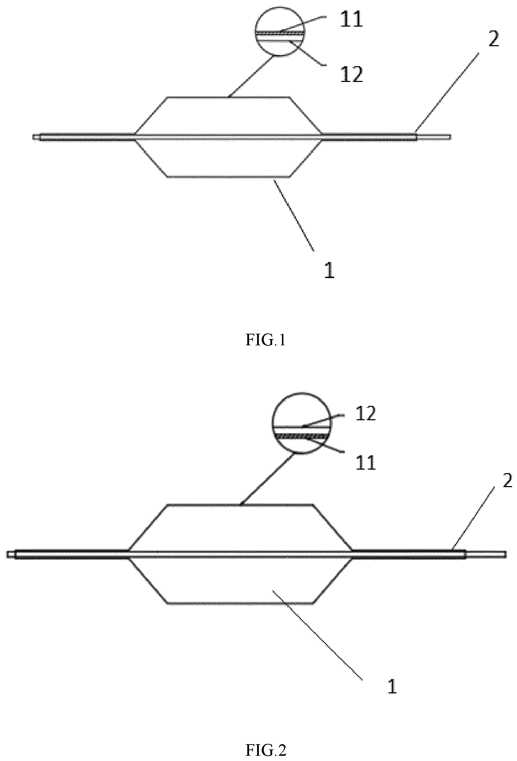

As shown in Fig. 1, this example provides a pulse balloon. The pulse balloon includes a balloon body 1 and an inner tube 2; the inner tube 2 penetrates through the balloon body 1; the inner tube 2 is located on the symmetry axis of the balloon body 1; the inner tube 2 is provided with an electrode; the balloon body 1 includes an insulating layer 11 and a balloon wall 12 from outside to inside; the balloon wall has a thickness of 20 µm, and the insulating layer has a thickness of 3 µm; the material of the balloon wall is polyethyleneimine, and the material of the insulating layer is Teflon.

### Example 2

As shown in Fig. 1, this example provides a pulse balloon. The pulse balloon includes a balloon body 1 and an inner tube 2; the inner tube 2 penetrates through the balloon body 1; the inner tube 2 is located on the symmetry axis of the balloon body 1; the inner tube 2 is provided with an electrode; the balloon body 1 includes an insulating layer 11 and a balloon wall 12 from outside to inside; the balloon body 1 is prepared by spraying; the balloon wall has a thickness of 15 µm, and the insulating layer has a thickness of 4 µm; the material of the balloon wall is polyethyleneimine, and the material of the insulating layer is Teflon.

The spraying includes: after the balloon wall is subjected to blow molding, a polyethyleneimine dispersion is sprayed onto the outer surface of the balloon wall, and dried at 190°C. When the pressure of the balloon reaches 14ATM, the pulse voltage is set to 5000V, and the discharging is started for 100 times at 1HZ. After repetition for 5 times, it is observed that no peeling, cracking, shrinkage and others occur between the insulating layer and the balloon wall.

### Example 3

As shown in Fig. 1, this example provides a pulse balloon. The pulse balloon includes a balloon body 1 and an inner tube 2; the inner tube 2 penetrates through the balloon body 1; the inner tube 2 is located on the symmetry axis of the balloon body 1; the inner tube 2 is provided with an electrode; the balloon body 1 includes an insulating layer 11 and a balloon wall 12 from outside to inside; the balloon body 1 is prepared by sleeve blow molding; the balloon wall has a thickness of 25 µm, and the insulating layer has a thickness of 2 µm; the material of the balloon wall is polyethyleneimine, and the material of the insulating layer is Teflon.

The sleeve blow molding includes: a PTFE film is sleeved on the outer layer of the balloon tube, put in the mold of the balloon molding machine for blow molding. The process parameters of blow molding are as follows: the blow molding temperature is 140°C, the heating time is 15s, the pressure of the left and right sides of the mold is 60 psi, the tensile speed is 130 min/s, and the cooling time of the left and right sides is 6s. When the pressure of the balloon reaches 14ATM, the pulse voltage is set to 5000V, and the discharging is started for 100 times at 1HZ. After repetition for 5 times, it is observed that no peeling, cracking, shrinkage and others occur between the insulating layer and the balloon wall.

### Example 4

As shown in Fig. 1, this example provides a pulse balloon. The pulse balloon includes a balloon body 1 and an inner tube 2; the inner tube 2 penetrates through the balloon body 1; the inner tube 2 is located on the symmetry axis of the balloon body 1; the inner tube 2 is provided with an electrode; the balloon body 1 includes an insulating layer 11 and a balloon wall 12 from outside to inside; the balloon body 1 is prepared by extrusion blow molding; the balloon wall has a thickness of 30 µm, and the insulating layer has a thickness of 2 µm; the material of the balloon wall is polyethyleneimine, and the material of the insulating layer is Teflon.

The extrusion blow molding includes: the insulating layer and the balloon wall are extruded into a complex tube with a tube extruder by outer diameter sizing, and putting the complex tube into the mold of the balloon molding machine for blow molding. The process parameters of blow molding are as follows: the blow molding temperature is 150°C, the heating time is 15s, the pressure of the left and right sides of the mold is 70 psi, the tensile speed is 150 mm/s, and the cooling time of the left and right sides is 10s. When the pressure of the balloon reaches 14ATM, the pulse voltage is set to 5000V, and the discharging is started for 100 times at 1HZ. After repetition for 5 times, it is observed that no peeling, cracking, shrinkage and others occur between the insulating layer and the balloon wall.

### Example 5

As shown in Fig.2, this example provides a pulse balloon. The pulse balloon includes a balloon body 1 and an inner tube 2; the inner tube 2 penetrates through the balloon body 1; the inner tube 2 is located on the symmetry axis of the balloon body 1; the inner tube 2 is provided with an electrode; the balloon body 1 includes an insulating layer 11 and a balloon wall 12 from outside to inside; the balloon wall has a thickness of 20 µm, and the insulating layer has a thickness of 3 µm; the material of the balloon wall is polyethyleneimine, and the material of the insulating layer is Teflon.

### Example 6

As shown in Fig.2, this example provides a pulse balloon. The pulse balloon includes a balloon body 1 and an inner tube 2; the inner tube 2 penetrates through the balloon body 1; the inner tube 2 is located on the symmetry axis of the balloon body 1; the inner tube 2 is provided with an electrode; the balloon body 1 includes an insulating layer 11 and a balloon wall 12 from outside to inside; the balloon body 1 is prepared by dipping; the balloon wall has a thickness of 25 µm, and the insulating layer has a thickness of 2 µm; the material of the balloon wall is polyethyleneimine, and the material of the insulating layer is Teflon.

The dipping includes: after blow molding of the balloon wall, the inner surface of the balloon wall is immersed into a polytetrafluoroethylene dispersion and then dried at 200°C after the polytetrafluoroethylene dispersion is discharged. When the pressure of the balloon reaches 14ATM, the pulse voltage is set to 5000V, and the discharging is started for 100 times at 1HZ. After repetition for 5 times, it is observed that no peeling, cracking, shrinkage and others occur between the insulating layer and the balloon wall.

### Example 7

As shown in Fig.2, this example provides a pulse balloon. The pulse balloon includes a balloon body 1 and an inner tube 2; the inner tube 2 penetrates through the balloon body 1; the inner tube 2 is located on the symmetry axis of the balloon body 1; the inner tube 2 is provided with an electrode; the balloon body 1 includes an insulating layer 11 and a balloon wall 12 from outside to inside; the balloon body 1 is prepared by extrusion blow molding; the balloon wall has a thickness of 25 µm, and the insulating layer has a thickness of 2 µm; the material of the balloon wall is polyethyleneimine, and the material of the insulating layer is Teflon.

The extrusion blow molding includes: the insulating layer and the balloon wall are extruded into a complex tube with a tube extruder by outer diameter sizing, and then put in the mold of the balloon molding machine for blow molding. The process parameters of blow molding are as follows: the blowing temperature is 150°C, the heating time is 13s, the pressure of the left and right sides of the mold is 70psi, the tensile speed is 140 mm/s, and the cooling time of the left and right sides is 20s. When the pressure of the balloon reaches 14ATM, the pulse voltage is set to 5000V, and the discharging is started for 100 times at 1HZ. After repetition for 5 times, it is observed that no peeling, cracking, shrinkage and others occur between the insulating layer and the balloon wall.

### Example 8

As shown in Fig.2, this example provides a pulse balloon. The pulse balloon includes a balloon body 1 and an inner tube 2; the inner tube 2 penetrates through the balloon body 1; the inner tube 2 is located on the symmetry axis of the balloon body 1; the inner tube 2 is provided with an electrode; the balloon body 1 includes an insulating layer 11 and a balloon wall 12 from outside to inside; the balloon body 1 is prepared by centrifugation; the balloon wall has a thickness of 20 µm, and the insulating layer has a thickness of 3 µm; the material of the balloon wall is polyethyleneimine, and the material of the insulating layer is Teflon.

The centrifugation includes: after blow molding of the balloon wall, the insulating layer material dispersion is introduced into the interior of the balloon wall, and subjected to centrifugal rotation at 170°C and 1500 rpm until the balloon wall is dried; when the pressure of the balloon reaches 14ATM, the pulse voltage is set to 5000V, and the discharging is started for 100 times at 1HZ. After repetition for 5 times, it is observed that no peeling, cracking, shrinkage and others occur between the insulating layer and the balloon wall.

### Example 9

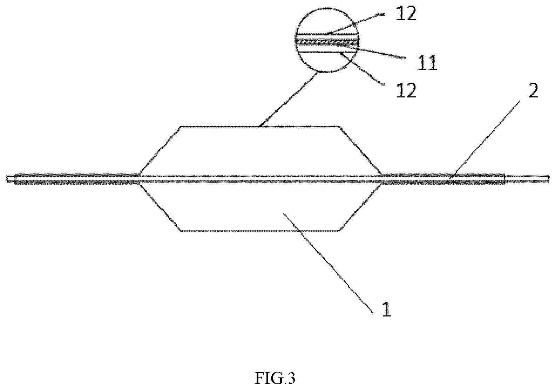

As shown in Fig.3, this example provides a pulse balloon. The pulse balloon includes a balloon body 1 and an inner tube 2; the inner tube 2 penetrates through the balloon body 1; the inner tube 2 is located on the symmetry axis of the balloon body 1; the inner tube 2 is provided with an electrode; the balloon body 1 includes an insulating layer 11 and a balloon wall 12; the insulating layer 11 is located inside the balloon wall 12; the balloon wall has a thickness of 20 µm (excluding the thickness of the insulating layer), and the insulating layer has a thickness of 3 µm; the material of the balloon wall is polyethyleneimine, and the material of the insulating layer is Teflon.

### Example 10

As shown in Fig.3, this example provides a pulse balloon. The pulse balloon includes a balloon body 1 and an inner tube 2; the inner tube 2 penetrates through the balloon body 1; the inner tube 2 is located on the symmetry axis of the balloon body 1; the inner tube 2 is provided with an electrode; the balloon body 1 includes an insulating layer 11 and a balloon wall 12; the insulating layer 11 is located inside the balloon wall 12; the balloon body 1 is prepared by extrusion blow molding; the balloon wall has a thickness of 20 µm (excluding the thickness of the insulating layer), and the insulating layer has a thickness of 3 µm; the material of the balloon wall is polyethyleneimine, and the material of the insulating layer is Teflon.

The extrusion blow molding includes: the insulating layer and the balloon wall are extruded into a complex tube with a tube extruder by outer diameter sizing, and then put in the mold of the balloon molding machine for blow molding. The process parameters of blow molding are as follows: the blowing temperature is 140°C, the heating time is 13s, the pressure of the left and right sides of the mold is 70psi, the tensile speed is 150mm/s, and the cooling time of the left and right sides is 20s. When the pressure of the balloon reaches 14ATM, the pulse voltage is set to 5000V, and the discharging is started for 100 times at 1HZ. After repetition for 5 times, it is observed that no peeling, cracking, shrinkage and others occur between the insulating layer and the balloon wall.

Examples 1-10 provide a pulse balloon, which can limit excess high voltage electricity at the pulse source, and prevent excess electricity from being conducted to the pacemaker, the signal conduction area and the like in the process of using the balloon, so that the patient's heart rate can maintained at a relatively stable state. Furthermore, the pulse balloon used in the present invention can be used under high voltage and discharging times, and has a high cracking effect on severe calcification.

The foregoing embodiments are illustrative only and serve to explain some of the features of the methods described herein. The appended claims are intended to claim the broadest conceivable scope and the embodiments presented herein are merely illustrative of selected implementations according to a combination of all possible embodiments. Accordingly, the applicant's intention is that the appended claims not be limited by the selection of examples that characterize the present invention. Some of the numerical ranges used in the claims also include sub-ranges within them, and variations within these ranges should also be construed, where possible, to be covered by the appended claims.

## Claims

1. A pulse balloon comprising a balloon body and an inner tube, wherein the inner tube penetrates through the balloon body; the balloon body comprises an insulating layer and a balloon wall, the insulating layer is located at one side and/or interior of the balloon wall; the balloon wall has a thickness of 10-30 µm, and the insulating layer has a thickness of 1-5 µm;
when the insulating layer is located at one side of the balloon wall, the balloon body is prepared by coating, centrifugation, sleeve blow molding or extrusion blow molding;
when the insulating layer is located inside the balloon wall or when the insulating layer is located at one side and the interior of the balloon, the balloon body is prepared by extrusion blow molding;
the coating comprises: after the balloon wall is subjected to blowing molding, coating an insulating layer material dispersion at one side of the balloon wall, and drying at 150-200°C;
the centrifugation comprises: after the balloon wall is subjected to blowing molding, introducing an insulating layer material dispersion into the interior of the balloon wall, and undergoing centrifugal rotation at 150-200°C;
the sleeve blow molding comprises: sheathing an insulating layer film outside a balloon tube for blow molding;
the extrusion blow molding comprises: co-extruding the insulating layer and the balloon wall to obtain a complex tube for blow molding;
the blow molding has process parameters as follows: the temperature is 100-250°C, the heating time is 10-150s, and the pressure is 50-1000psi.

2. The pulse balloon according to claim 1, **characterized in that** the balloon body comprises the insulating layer and the balloon wall from outside to inside.

3. The pulse balloon according to claim 1, **characterized in that** the balloon body comprises the balloon wall and the insulating layer from outside to inside.

4. The pulse balloon according to claim 1, **characterized in that** the insulating layer is located inside the balloon wall.

5. The pulse balloon according to claim 1, **characterized in that** the inner tube is in seal connection with the balloon body.

6. The pulse balloon according to claim 1, **characterized in that** the inner tube is located on the symmetry axis of the balloon body.

7. The pulse balloon according to claim 1, **characterized in that** the inner tube is provided with an electrode.

8. The pulse balloon according to claim 1, **characterized in that** the material of the balloon wall is selected from one or more of nitrogenous polymers, polyesters and epoxy resins.

9. The pulse balloon according to any one of claims 1-8, **characterized in that** the material of the insulating layer is selected from one or more of plastics, rubbers, fibers and inorganic materials.

10. Use of the pulse balloon according to any one of claims 1-9 for shielding electrical interference in a vascular calcification area.
